# EUROPÄISCHE PATENTSCHRIFT

(11) **EP 1 937 227 B1**
(45) Veröffentlichungstag und Bekanntmachung des Hinweises auf die Patenterteilung: **15.04.2009**
(21) Anmeldenummer: 06806011.0
(22) Anmeldetag: 04.10.2006
(51) Int. Cl.: A61K 9/70, A61K 31/00

(54) **TRANSDERMALES THERAPEUTISCHES SYSTEM ZUR VERABREICHUNG LIPOPHILER UND/ODER WENIG HAUTPERMEABLER WIRKSTOFFE**
TRANSDERMAL THERAPEUTIC SYSTEM FOR ADMINISTERING LIPOPHILIC AND/OR CUTANEOUS LOW-PERMEABLE ACTIVE SUBSTANCES
SYSTEME THERAPEUTIQUE TRANSDERMIQUE POUR ADMINISTRER DES SUBSTANCES ACTIVES LIPOPHILES ET/OU A FAIBLE PERMEABILITE CUTANEE

(30) Priorität: 21.10.2005 DE 102005050431
(43) Veröffentlichungstag der Anmeldung: 02.07.2008
(73) Patentinhaber: LTS LOHMANN Therapie-Systeme AG, 56626 Andernach (DE)
(72) Erfinder: KOCH, Andreas, 56581 Melsbach (DE); SCHMITZ, Christoph, 56598 Rheinbrohl (DE); PRACHT, Rolf, 56203 Höhr-Grenzhausen (DE); SAMETI, Mohammad, 53177 Bonn (DE)
(74) Vertreter: Flaccus, Rolf-Dieter
(86) Internationale Anmeldenummer: PCT/EP2006/009570
(87) Internationale Veröffentlichungsnummer: WO 2007/045352

(56) Entgegenhaltungen:
- WO-A-00/35456
- DE-A1- 19 701 059

## Beschreibung

Die vorliegende Erfindung betrifft ein transdermales therapeutisches System zur Verabreichung von lipophilen, schlecht wasserlöslichen und/oder wenig hautpermeablen pharmazeutischen Wirkstoffen, insbesondere ein transdermales therapeutisches Flüssigreservoir-System zur Verabreichung von Vinpocetin, Moxonidin, Pergolid oder eines ihrer pharmazeutisch akzeptablen Salze.

Vinpocetin (Ethylapovincamin-22-oat; 3α,16α-Apovincaminsäureethylester; Ethyl-12-desoxy-12,13-didehydro-vincanol-12-carboxylat) (CAS-Nr. 42971-09-5) ist ein pharmazeutischer Wirkstoff mit antioxidativer, gefäßerweiternder und neuroprotektiver Wirkung, der zur symptomatischen Behandlung von chronischen, hirnorganisch bedingten Leistungsstörungen in Form von Gedächtnis-, Konzentrations-, Denk- und Affektstörungen, vorzeitiger Ermüdbarkeit sowie Antriebs- und Motivationsmangel eingesetzt wird. Gegenwärtig befindet sich dieser Wirkstoff ausschließlich als orale Darreichungsform (Tablette) mit 20 mg Vinpocetin in Form seiner freien Base unter dem Handelsnamen Cavinton^{®} auf dem Markt. Allerdings beträgt die Resorptionsquote von Vinpocetin im nüchternen Zustand nur etwa 6 bis 7 Prozent. Bei Einnahme während der Mahlzeiten verbessert sich die Resorption um 60 bis 100 Prozent. In beiden Fällen gelangt Vinpocetin etwa eine Stunde nach der Verabreichung in die Blutbahn. Die Eliminationshalbwertszeit bei oraler Verabreichung beträgt ein bis zwei Stunden; innerhalb von acht Stunden-wird Vinpocetin nahezu vollständig aus dem Organismus ausgeschieden. Zusammenfassend ist festzustellen, dass die Bioverfügbarkeit oral verabreichten Vinpocetins sehr schlecht ist.

Zur Verbesserung der Bioverfügbarkeit von Vinpocetin käme eine transdermale Verabreichung mit Hilfe eines transdermalen therapeutischen Systems in Betracht. Allerdings hat Vinpocetin für eine transdermale Verabreichung ungünstige physikochemische Eigenschaften. So charakterisieren der Verteilungskoeffizient LogP_{Octanol/Wasser} von etwa 3,9 und die schlechte Wasserlöslichkeit von 0,02 µg/ml bei Raumtemperatur Vinpocetin als sehr lipopilen Arzneiwirkstoff.

Kobayashi, D. et al. (Biol. Pharm. Bull., 16, (19939, 254-258) beschreiben, dass mit einer Wirkstoff-Enhancermischung in Form einer 1 %-igen Suspension von Vinpocetin in einer Mischung aus Ethanol und 1-Mentol, die in-vitro direkt auf humane Vollhaut aufgetragen wurde, eine Permeationsrate von 6,45 µg/cm² x h erreicht werden kann. Allerdings ermöglicht diese flüssige Zubereitung keine anhaltende Wirkstofffreisetzung, sondern muss wiederholt aufgetragen werden, um einen annähernd konstanten Plasmaspiegel an Vinpocetin über den Tag zu erzielen. Daher dürfte eine derartige flüssige Darreichungsform wenig Akzeptanz bei Patienten finden.

DE 197 01 059 A1 offenbart ein Acetylsalicylsäure enthaltendes transdermales therapeutisches System mit einer im wesentlichen wirkstoff- und feuchtigkeitsundurchlässigen Rückschicht, einem Wirkstoffreservoir und gegebenenfalls einer wiederablösbaren Schutzschicht. Eingesetzt wird eine Kombination aus Penetrationsverbesserern, nämlich aus Limonen und Dimethylisosorbid, um einen höheren Wirkstoffanteil durch die Hautschichten dringen zu lassen.

WO 00/35456 beschreibt ein Buprenorphin enthaltendes transdermales therapeutisches System, das als Penetrationsverbesserer Terpene (L-Menthol, Menthon, D-Limonen, 1,8-Cineol, Nerolidol, Carveol, Campher), C8-C18 aliphatische Alkohole (wie Laurylalkohol), Glycerolmonolaurat oder Glyceroldilaurat oder Glyceroltrilaurat / Glyceroltricaprilat / Glyceroltricaprat.

Erste Voruntersuchungen der Anmelderin im Hinblick auf die Entwicklung eines Matrix-TTS zur Verabreichung von Vinpocetin führten zu transdermalen therapeutischen Systemen (TTS) mit denen ein Hautflux (Permeationsrate im

Fließgleichgewicht) von 0,91 µg/cm² x h erreicht werden konnte. Um jedoch die therapeutisch erforderliche Konzentration von Vinpocetin im Plasma eines Patienten auf 2 bis 3 ng/ml einzustellen, müsste ein für eine Anwendungsdauer von 24 Stunden ausgelegtes TTS eine Fläche von etwa 120 cm² aufweisen. Aus Gründen der Akzeptanz beim Patienten sollte die Fläche eines TTS jedoch so klein wie möglich gehalten werden und nach Möglichkeit 40 cm², vorzugsweise 20 cm², nicht übersteigen.

Die der vorliegenden Erfindung zunächst zugrunde liegende Aufgabe bestand somit in der Bereitstellung eines transdermalen therapeutischen Systems zur Verabreichung von Vinpocetin, das eine akzeptable Größe von nicht mehr als 40 cm² hat. Anders ausgedrückt bestand die Aufgabe darin, ein TTS zur Verabreichung von Vinpocetin zu entwickeln, mit dem eine Permeationsrate von mindestens 2,15 µg/cm² x h, vorzugsweise von mindestens 5,0 µg/cm² x h, erreicht werden kann, was einer transdermalen Abgaberate von mindestens 2 mg/Tag entspricht. Mit einer derart hohen Abgaberate bzw. Tagesdosis könnte der therapeutisch notwendige Plasmaspiegel in Höhe von 2 bis 3 ng Vinpocetin pro Milliliter Plasma erreicht werden.

Diese Aufgabe wird überraschenderweise durch ein Flüssigreservoir-System gelöst, bei dem Vinpocetin in einem Mehrkomponenten-Enhancersystem gelöst oder suspendiert vorliegt, wobei das Enhancersystem aus 4 Permeationsenhancern (= Komponenten des Enhancersystems) besteht und jede Komponente des Enhancersystems aus einer anderen der folgenden Stoffgruppen ausgewählt ist:
a) Terpene
b) cyclische Glucitol-Ether,
c) mittelkettige Triglyceride aus Caprin- und Caprylsäure und/oder Linolsäure; und
d) längerkettige Alkohole.

Im Rahmen weiterer Untersuchungen der Anmelderin hat sich überraschenderweise herausgestellt, dass sich das erfindungsgemäße Enhancersystem auch in Verbindung mit anderen Wirkstoffen, die dem Vinpocetin in seinen physikochemischen Eigenschaften ähnlich sind, d. h. bei denen es sich um lipophile, schlecht wasserlösliche und/oder wenig hautpermeable Wirkstoffe handelt, vorteilhaft nutzen lässt.

Die vorliegende Erfindung betrifft somit transdermale therapeutische Systeme zur Verabreichung von lipophilen, schlecht wasserlöslichen und/oder wenig hautpermeablen pharmazeutischen Wirkstoffen.

"Lipophil" im Sinne der vorliegenden Beschreibung bedeutet, dass der Wirkstoff ein großes Bestreben hat, in eine mit Wasser nicht mischbare Phase überzugehen. Lipophile Substanzen zeichnen sich durch einen hohen LogP-Wert aus. Der LogP-Wert ist der Verteilungskoeffizient einer Substanz zwischen Octanol und Wasser: C_{Octanol}/C_{Wasser}.

Unter "schlecht wasserlöslich" werden im Sinne der vorliegenden Beschreibung Wirkstoffe verstanden, die eine Löslichkeit von weniger als 0,3 Gew.-% in Wasser aufweisen, d.h. Substanzen, von denen weniger als 3 mg in einem Milliliter Wasser löslich sind.

"Wenig hautpermeabel" im Sinne der vorliegenden Beschreibung bezeichnet Wirkstoffe, deren Flux allein, d. h. ohne Permeationsverstärker oder andere Maßnahmen zur Erhöhung der Hautdurchdringung, zu gering ist, um die zur Erzielung eines therapeutischen Effekts erforderliche Wirkstoffkonzentration im Plasma erzielen zu können. Im Allgemeinen werden Wirkstoffe, die ein Molekulargewicht > 500 oder einen LogP von weniger als -1 oder mehr als 4 aufweisen, als wenig hautpermeabel angesehen.

Lipophile, schlecht wasserlösliche Wirkstoffe zeichnen sich in der Regel bei oraler Verabreichung durch eine schlechte Bioverfügbarkeit aus. Zwar ist eine transdermale Verabreichung von Wirkstoffen, die bei oraler Verabreichung schlecht bioverfügbar sind, dem Grunde nach denkbar, aber lipophile Wirkstoffe gehören üblicherweise auch zu den wenig bzw. schlecht hautpermeablen Wirkstoffen.

Ein bevorzugter Wirkstoff, der mit Hilfe des erfindungsgemäßen Enhancersystems mit einer für therapeutische Zwecke ausreichenden Permeationsrate über die Haut verabreicht werden kann, ist Moxonidin.

Moxonidin (4-Chlor-N-(4,5-dihydro-1H-imidazol-2-yl)-6-methoxy-2-methyl-5-pyrimidinamin; 4-Chlor-6-methoxy-2-methyl-5-(2-imidazolin-2-yl)aminopyrimidin) (CAS-Nr. 75438-57-2) ist ein Imidazolderivat, das zur Behandlung der essentiellen Hypertonie empfohlen wird. Es ist als Cynt^{®}**-** oder Physiotens^{®}**-**Filmtabletten erhältlich. Moxonidin wird gastrointestinal gut resorbiert und präsystemisch nicht metabolisiert, so dass seine biologische Verfügbarkeit bei etwa 90% liegt. Moxonidin hat jedoch eine Plasma-Halbwertszeit von nur 2 bis 3 Stunden und wird im Wesentlichen über die Nieren ausgeschieden. Die benötigte Tagesdosis beträgt etwa 0,2 bis 0,6 mg; der wirksame Plasmaspiegel liegt bei 5,4 ng/ml (nach Farsang, C., J. Clin. Basic Cardiol. 4, (2001), 197). Eine vorteilhafte Wirkung des Moxonidins ist dessen Senkung der gesteigerten Aktivität des Sympathikotonus bei Niereninsuffizienz. Allerdings muss im Vergleich zur Dosis eines Nierengesunden die Erhaltungsdosis von Moxonidin bei Niereninsuffizinez erniedrigt werden und/oder das Dosierungsintervall verlängert werden.

Von den klinisch nützlichen Blutdruck senkenden Wirkstoffen hat Moxonidin die niedrigste Basizität. Bei einem pH-Wert von 7,4 liegen nur etwa 50 % des Moxonidins in kationischer Form vor. Moxonidin ist ein lipophiler Wirkstoff; er weist eine sehr geringe Löslichkeit in Wasser auf, ist jedoch bis 100 mM in Ethanol oder DMSO löslich. Die Wasserlöslichkeit von Moxonidin (Base) beträgt 1,36 mg/ml bei 22 °C. Der Verteilungskoeffizient LogP_{Octanol/Wasser} liegt bei -1,3. Diese beiden Werte charakterisieren Moxonidin (die freie Base) als schlecht wasserlösliche und wenig hautpermeable Substanz.

Ein weiterer Wirkstoff, der mit Hilfe des erfindungsgemäßen Enhancersystems mit einer für therapeutische Zwecke ausreichenden Permeationsrate über die Haut verabreicht werden kann, ist Pergolid.

Pergolid ((8β)-8-[(Methylthio)methyl]-6-propylergolin, D-6-n-propyl-8β-methylmercaptomethylergolin) (CAS-Nr. 66104-22-1) ist ein semisynthetisches Ergolin mit starker Dopamin(D)-2- und geringerer D-1-Rezeptor-agonistischer Wirkung, das zur Behandlung der Parkinson-Krankheit zugelassen ist. Pergolid ist chemisch zwar anders gebaut als Dopamin, übt seine Wirkung aber an den gleichen Bindungsstellen wie Dopamin aus. Darüber hinaus hat es auch eine die Prolaktinkonzentration im Plasma senkende Wirkung. Die Plasma-Halbwertszeit von Pergolid nach oraler Verabreichung beträgt 27 Stunden. Die Tagesdosis beträgt etwa 5 mg (Gerschlager, W., J. Neurol. Neurochem. Psychiatr., 5, (2004), 21-24). Die Wasserlöslichkeit von Pergolid (Base) beträgt 0,021 µg/ml bei 22 °C. Der Verteilungskoeffizient LogP_{Octanol/Wasser} liegt bei 4,02. Diese beiden Werte charakterisieren die Pergolid-Base als eine sehr schlecht wasserlösliche und wenig hautpermeable Substanz.

Die pharmakologisch akzeptablen Salze des Vinpocetins, Moxonidins und Pergolids zählen aufgrund ihres ionischen Charakters ebenfalls zu den wenig hautpermeablen Substanzen, auch wenn deren Wasserlöslichkeit größer ist als die des Vinpocetins, Moxonidins bzw. Pergolids. Daher gehören auch die pharmakologisch akzeptablen Salze des Vinpocetins, die pharmakologisch akzeptablen Salze des Moxonidins und die pharmakologisch akzeptablen Salze des Pergolids zu den Substanzen bzw. Wirkstoffen, die mit dem erfindungsgemäßen Mehrkomponenten-Enhancersystem mit für therapeutische Zwecke ausreichender Permeationsrate transdermal verabreicht werden können. Beispiele für bekannte Salze der genannten Wirkstoffe sind das Moxonidin-Hydrochlorid und das Pergolid-Mesylat.

Transdermal therapeutische Flüssigreservoir-Systeme an sich, bei denen eine in der Regel flüssige Wirkstoffzubereitung in einem aus einer die Freisetzung steuernden Membran und einer vorzugsweise wirkstoffundurchlässigen Folie gebildeten Beutel enthalten ist, sind dem Fachmann bekannt. Bei diesen Systemen dienen pharmazeutisch akzeptable und hautverträgliche organische Lösemittel als Trägermedium, dessen Viskosität mit geeigneten Hilfsstoffen (z.B. Mineralölen) auf die jeweiligen technologischen Erfordernisse eingestellt werden kann. Im Idealfall haben die verwendeten Lösemittel gleichzeitig die Permeation des Wirkstoffs durch die Haut des Patienten fördernde Eigenschaften. Dem Lösemittel können aber auch die Permeation des Wirkstoffs fördernde Substanzen, so genannte Enhancer, zugesetzt werden. Darüber hinaus umfassen die Flüssigreservoir-Systeme eine Haftkleberschicht, mit der das System auf der Haut des Patienten befestigt wird.

Bei dem erfindungsgemäßen Flüssigreservoir-System liegt der lipophile, schlecht wasserlösliche und/oder wenig hautpermeable Wirkstoff in einem Mehrkomponenten-Enhancersystem gelöst, dispergiert oder suspendiert vor, wobei dieses Enhancersystem aus vier die Permeation des Wirkstoffs verstärkenden Enhancern besteht. Dabei stammt jeder Enhancer des Mehrkomponenten-Enhancersystems aus einer anderen Stoffgruppe. Die Stoffgruppen, aus denen die Enhancer strammen, sind:
a) Terpene;
b) cyclische Glucitol-Ether;
c) Triglyceride aus Caprin- und Caprylsäure und/oder Linolsäure; und
d) längerkettige Alkohole.

Terpene lassen sich vom Isopren bzw. von Isopentenyleinheiten ableiten, so dass sich ihre Kohlenstoffanzahl im Grundgerüst durch 5 teilen lässt. Nach Anzahl der Isopren-Reste unterscheidet man Monoterpene (C₁₀), Sesquiterpene (C₁₅), Diterpene (C₂₀), Sesterterpene (C₂₅), Triterpene (C₃₀), Tetraterpene (C₄₀) und Polyterpene. Darüber hinaus gibt es auch natürlich vorkommnende Terpene, deren Struktur nicht mit der Isopren-Regel in Einklang steht. Bei den Terpenen handelt es sich um eine heterogene Gruppe von Verbindungen, die aufgrund ihrer unterschiedlichen funktionellen Gruppen zu verschiedenen Stoffgruppen zugeordnet werden können, so dass man unter Terpenen sowohl die Kohlenwasserstoffe als auch die sich davon ableitenden Alkohole, Ketone, Aldehyde und Ester versteht.

Erfindungsgemäß bevorzugt werden Monoterpene, d. h. aus zwei Isopren-Einheiten aufgebaute Terpene. Beispiele für Monoterpene sind Pinen, Nerol, Citral, Campher, Menthol, Carvacrol, Thymol und Limonen.

Die Monoterpene können in acyclische, monocyclische und bicyclische Monoterpene unterteilt werden. Zu den acyclischen Monoterpenen gehören beispielsweise Myrcen, Ocimen und Cosmen. Besonders bevorzugt werden erfindungsgemäß die monocyclischen Monoterpene, die ein Cyclohexangerüst aufweisen, sich also vom p-Menthan ableiten lassen.

Die monocyclischen Monoterpene mit Cyclohexangerüst werden meist wiederum nach ihrer sekundären Stoffgruppenzugehörigkeit unterteilt. Ganz besonders bevorzugt werden die alkoholischen monocyclischen Monoterpene mit Cyclohexangerüst, insbesondere Eucalyptol.

Als cyclischer Glucitol-Ether ist insbesondere Dimethylisosorbid geeignet. Glucitol, das auch Sorbitol genannt wird, ist der Zuckeralkohol der Glucose.

Bei den Triglyceriden aus Caprin- und Caprylsäure und/oder Linolsäure werden bevorzugt die unter dem Handelsnamen Miglyol vertriebenen Triglyceride verwendet, vorzugsweise Miglyol 808, Miglyol 810 und Miglyol 812, wobei Miglyol 812 besonders bevorzugt wird.

Als längerkettige Alkohole werden Alkohole mit einer Kettenlänge von mindestens 8 Kohlenstoff-Atomen, vorzugsweise mit mindestens 12 Kohlenstoff-Atomen, verwendet, vorzugsweise einwertige Alkohole, besonders bevorzugt 1-Dodecanol.

In einer bevorzugten Ausführungsform liegen die Komponenten des Mehrkomponenten-Enhancersystem in einem Mischungsverhältnis vor, bei dem der Anteil der am niedrigsten dosierten Komponente mindestens 10 Gew.-% und der Anteil der am höchsten dosierten Komponente maximal 40 Gew.-%, jeweils bezogen auf das Gesamtgewicht des Mehrkomponenten-Enhancersystems, beträgt.

Das am stärksten bevorzugte Mehrkomponenten-Enhancersystem setzt sich wie folgt zusammen:
a) 25 Gew.-% Eucalyptol als Terpen;
b) 25 Gew.-% Dimethylisosorbid als cyclischer Glucitol-Ether;
c) 35 Gew.-% Miglyol 812 als mittelkettiges Triglycerid von Capryl- und Caprinsäure; und
d) 15 Gew.-% 1-Dodecanol als längerkettiger Alkohol mit einer Kettenlänge von 12 oder mehr Kohlenstoffatomen.

Der Anteil an Wirkstoff in der wirkstoffhaltigen Zubereitung beträgt 0,1 bis 50 Gew.-%, vorzugsweise 5 bis 25 Gew.-%.

Die im Flüssig-Reservoir enthaltene Zubereitung umfasst somit den Wirkstoff, der in dem Mehrkomponenten-Enhancersystem gelöst, dispergiert oder suspendiert vorliegen kann. Darüber hinaus kann die WirkstoffZubereitung weitere Substanzen enthalten, mit denen die Abgabe des Wirkstoffs - im Sinne einer verzögerten Abgabe - gesteuert werden kann. Derartige Substanzen sind beispielsweise Absorptionsmittel.

Die Absorptionsmittel können aus der Gruppe ausgewählt sein, die Cyclodextrine, Polyvinylpyrrolidone und Cellulosederivate enthält.

Die Wirkstoffzubereitung kann zusätzlich viskositätserhöhende Hilfsstoffe enthalten, die keine die Freisetzung steuernde Funktion haben. Vorzugsweise ist der viskositätserhöhende Hilfsstoff aus der Gruppe ausgewählt, die aus feindispersem Siliziumdioxid, beispielsweise Aerosil R 974^{®}, Polyacrylsäuren, z. B. Carbopol 934P^{®}**,** Mineralölen, Wollwachsen und hochmolekularen Polyethylenglykolen besteht. Ein Beispiel für ein bevorzugtes Polyethylenglykol ist Carbowax 1000^{®}. Die Wirkstoffzubereitung im Flüssigreservoir kann als Lösung, Dispersion, Suspension, Paste oder Gel vorliegen.

Bei dem erfindungsgemäßen TTS kann die Freisetzung des Wirkstoffs und der Enhancer aus dem Flüssig-Reservoirsystem kontrolliert werden über:
- die Art der eingesetzten Steuermembran, beispielsweise durch deren chemische Zusammensetzung und/oder der Porengröße;
- die Art der eingesetzten Haftkleberschicht unter der Steuermembran, mit der das System auf der Haut befestigt wird, beispielsweise durch deren chemische Zusammensetzung und/oder Schichtdicke;
- eine verzögerte Abgabe durch den Einsatz von Absorptionsmitteln im Flüssigreservoir, beispielsweise Cyclodextrinen, Polyvinylpyrrolidonen oder Cellulosederivaten.

Als Steuermembran können mikroporöse, mit definierter Porengröße hergestellte Polymerfolien aus Polyethylen, Polypropylen, Polyurethan, Copolymeren aus Ethylen und Vinylacetat und Silikonen verwendet werden. Geeignet sind diese Polymerfolien, sofern sie beständig gegenüber den in der Wirkstoffzubereitung enthaltenen Substanzen sind.

Als Haftkleber mit die Freisetzung des pharmazeutischen Wirkstoffs steuernden Eigenschaften, die unter der Steuermembran zur Befestigung des Systems auf der Haut angebracht sind, werden Haftkleber auf Basis von Copolymeren aus Ethylen und Vinylacetat, in Kombination mit Klebharzen als Zusätze, bevorzugt. Bei diesen Haftklebern kann die Durchlässigkeit bzw. Permeabilität der Haftkleberschicht über das Verhältnis von Ethylen zu Vinylacetat eingestellt werden. Bevorzugt werden auch Haftkleber auf Basis von Silikonen, da diese für die meisten Wirk- und Hilfsstoffe permeabel sind, sowie Haftkleber auf Basis von Poly(meth)acrylaten und Haftkleber aus Basis von Polyisobutylenen.

Nachfolgend wird die Erfindung an Hand von Beispielen näher erläutert, wobei diese Beispiele nicht als die einzigen Kombinationen zu verstehen sind, für die mit dieser Schrift Schutz begehrt wird.

### Beispiele 1 bis 4

Für die Herstellung der haftklebenden Flüssigreservoir-Systeme, wie sie in Tabelle 1 näher spezifiziert sind, wurde zunächst eine wirkstofffreie Polyacrylat-Kleberlösung vom Typ DuroTak^{®} 1050 (Fa. National Starch, Antwerpen) oder ein Copolymer aus Ethylen und Vinylacetat mit Zusatz eines Klebharzes auf Basis von Kolophonium (Foral^{®} 85 B) mit Hilfe einer Ausziehrakel in einer Nassschichtdicke von 300 µm auf einer silikonisierten Polyethylenterephthalat-Folie ausgestrichen. Anschließend wurden die Lösemittel durch halbstündiges Trocknen der beschichteten Folie bei 50 °C in einem Trockenschrank mit Abluftführung entfernt. Der lösemittel- und wirkstofffreie Klebefilm wurde danach mit einer 35 µm dicken Polyurethanfolie (Opraflex^{®}, Fa. Lohmann) oder einer Polypropylenfolie als spätere Steuermembran durch Aufkaschieren abgedeckt. Auf die Steuermembran wurde eine Polyesterfolie (Scotchpak^{®} No. 1220, Fa. 3M) aufgelegt und mit einer speziellen Siegelmaske, die über ein handelsübliches Bügeleisen aufgeheizt wurde, zu Beuteln gesiegelt, die ein rundes Reservoir mit einem Durchmesser von 25 mm aufwiesen.

Über eine vorhandene Öffnung zum Reservoir wurde die jeweilige Vinpocetin-Enhancermischung - die Enhancer-Komponenten wurden vorher in den entsprechenden Gewichtsanteilen zusammengemischt - mittels einer Spritze in das Reservoir gefüllt. Die Menge an Vinpocetin betrug 10 Gew.-%, bezogen auf das Gesamtgewicht der Wirkstoff-Enhancermischung. Nach dem Befüllen des Reservoirs wurde die Einfüllöffnung mit Hilfe eines Bügeleisens verschweißt, so dass ein vollständig abgeschlossenes und lagerstabiles Flüssig-Reservoirsystem entstand.

### Vergleichsbeispiel 1

Als Referenz für die Vinpocetin enthaltenden Flüssigreservoir-Systeme wurde ein Matrix-TTS hergestellt, indem Oppanol^{®} B 10 und Oppanol**^{®}** B 100, mittel- und hochmolekulare Polyisobutylene der Fa. BASF, in unterschiedlichen Mischungsverhältnissen in Spezialbenzin Typ 80/110 gelöst und anschließend 2 Gew.-% Vinpocetin und 17 Gew.-% Lauryllactat als Enhancer darin dispergiert. Durch 30-minütiges Rühren wurde eine gleichmäßige Verteilung der Wirkstoffkristalle in der Kleberlösung erreicht. Danach erfolgte eine 10 bis 15-minütige Entgasung der Dispersion im Ultraschallbad.

Für die Herstellung des selbstklebenden, wirkstoffhaltigen Films wurde eine silikonisierte Polyethylenterephthalat-Folie mit Hilfe einer Handrakel mit der wirkstoffhaltigen Klebmasse in einer Nassschichtdicke von 400 µm beschichtet. Das Ablüften des Kleberstriches erfolgte für 20 Minuten bei Raumtemperatur, bevor der Strich für 10 Minuten bei 70 °C getrocknet wurde.

Die lösemittelfreie, wirkstoffhaltige und selbstklebende Beschichtung wurde abschließend mit einer 15 µm dicken Polyethylenterephthalat-Folie durch Aufkaschieren abgedeckt.

### Beispiele 5 und 6

Haftklebende Flüssigreservoirsysteme zur Verabreichung von Moxonidin wurden entsprechend dem für die Beispiele 1 bis 4 angegebenen Verfahren hergestellt, indem eine wirkstofffreie Polyacrylat-Kleberlösung nach dem Ausstreichen und Trocknen mit einer 35 µm dicken Polyurethanfolie (Opraflex^{®}, Fa. Lohmann) als Steuermembran durch Aufkaschieren abgedeckt wurde. Auf die Steuermembran wurde eine Polyesterfolie aufgelegt und mit der speziellen Siegelmaske zu Beuteln mit einem runden Reservoir gesiegelt, welches einen Durchmesser von 25 mm aufwies.

Über eine vorhandene Öffnung zum Reservoir wurde die jeweilige, 10 Gew.-% Moxonidin enthaltende Wirkstoff-Enhancermischung mittels einer Spritze in das Reservoir gefüllt, welches anschließend verschweißt wurde, so dass ein vollständig geschlossenes und lagerstabiles Flüssig-Reservoirsystem entstand.

### Vergleichsbeispiel 2

Als Vergleichsbeispiel für die Moxonidin enthaltenden Flüssigreservoir-Systeme wurde ein Matrix-TTS hergestellt, das ein für die transdermale Verabreichung von Moxonidin bereits optimiertes TTS mit Permeationsenhancer darstellte.

### Beispiele 7 und 8

Haftklebende Flüssigreservoirsysteme zur Verabreichung von Pergolid wurden entsprechend dem für die Beispiele 1 bis 4 angegebenen Verfahren hergestellt, indem eine wirkstofffreie Polyacrylat-Kleberlösung nach dem Ausstreichen und Trocknen mit einer 35 µm dicken Polyurethanfolie (Opraflex^{®}, Fa. Lohmann) als Steuermembran durch Aufkaschieren abgedeckt wurde. Auf die Steuermembran wurde eine Polyesterfolie aufgelegt und mit der speziellen Siegelmaske zu Beuteln mit einem runden Reservoir gesiegelt, welches einen Durchmesser von 25 mm aufwies.

Über eine vorhandene Öffnung zum Reservoir wurde die jeweilige, 10 Gew.-% Pergolid enthaltende Wirkstoff-Enhancermischung mittels einer Spritze in das Reservoir gefüllt, welches anschließend verschweißt wurde, so dass ein vollständig geschlossenes und lagerstabiles Flüssig-Reservoirsystem entstand.

### Vergleichsbeispiel 3

Als Vergleichsbeispiel für die Pergolid enthaltenden Flüssigreservoir-Systeme wurde ein Matrix-TTS hergestellt, das ein für die transdermale Verabreichung von Pergolid bereits optimiertes TTS mit Permeationsenhancer darstellte.

### Besetzung der Permeationsraten

Die Permeationsraten der vorgenannten Systeme wurden am in-vitro-Diffusionsmodell der "Human-Epidermis" mit Hilfe modifizierter Franz-Diffusionszellen bestimmt. Als Akzeptormedium diente in allen Fällen Phosphatpuffer (pH 5,5) mit Zusatz von 0,1 % NaN₃ als Konservierungsmittel, thermostatiert auf 32 °C.

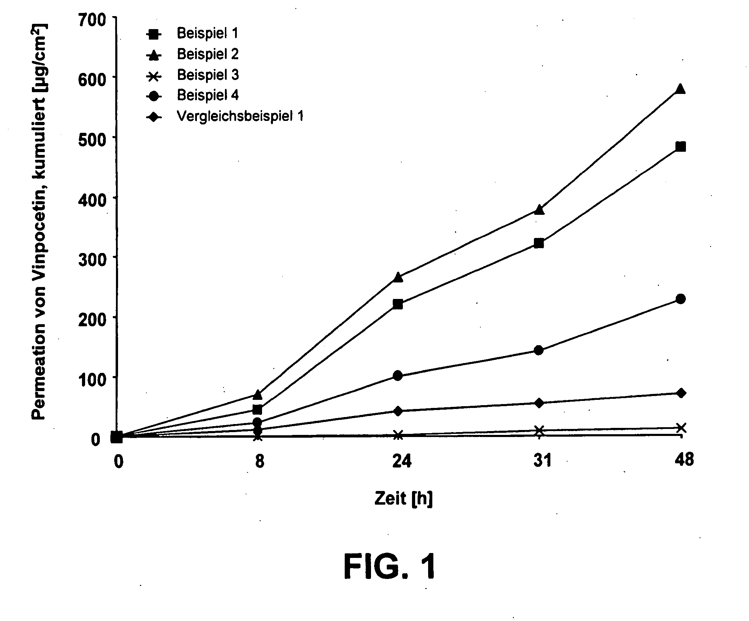

Die kumulierten Permeationsraten von Vinpocetin werden in Figur 1 dargestellt, wobei die einzelnen Messpunkte den Mittelwert aus drei Einzelmessungen darstellen. Die Ergebnisse der Untersuchungen zur Permeation von Vinpocetin sind in Tabelle 1 zusammengefasst.

**Tabelle 1: Vergleich der mit verschiedenen transdermalen therapeutischen Systemen erzielten Permeationsraten von Vinpocetin**

| System | Steuermembran | Haftkleber | Enhancer-Komponente(n) | Gew.-% der Komponenten im Enhancergemisch | In vitro-Hautflux [µg/cm²xh] |
|---|---|---|---|---|---|
| Beispiel 1 | Polyurethan | Polyacrylat | Miglyol Typ 812 | 35 | 10,9 |
| | | | Dimethylisosorbid | 25 | |
| | | | Eucalyptol | 25 | |
| | | | 1-Dodecanol | 15 | |
| | | | | | |
| Beispiel 2 | Polypropylen | Ethylen- | Miglyol Typ 812 | 35 | 13,0 |
| | | Vinylacetat- | Dimethylisonorbid | 25 | |
| | | Copolymer mit | Eucalyptol | 25 | |
| | | Zusatz eines | 1-Dodecanol | 15 | |
| | | Klebharzes auf | | | |
| | | Basis von | | | |
| | | Rollophonium | | | |
| | | | | | |
| Beispiel 3 | Polyurethan | Polyacrylat | Miglyol Typ 812 | 35 | 0,426 |
| | | | Dimethylisosorbid | 25 | |
| | | | Ethanol | 25 | |
| | | | 1-Dodecanol | 15 | |
| | | | | | |
| Beispiel 4 | Polyurethan | Polyacrylat | Lauryllactat | 100 | 1,16 |
| | | | | | |
| Vergleichs | N/A | Polyisobutylen | Lauryllactat | 17 | 1,10 |
| beispiel 1 | | | | | |

Aus Tabelle 1 ergibt sich beispielsweise für ein transdermales therapeutisches System gemäß Beispiel 1, dass entsprechend der Beziehung Q_{SS} = J_{SS} x A/Cl ein TTS mit einer 10 cm² großen Abgabefläche ausreichen würde, um einen Vinpocetin-Plasmaspiegel von 2,5 ng/ml erreichen zu können, wobei die Größen Q_{SS,} J_{SS}, A und Cl folgende Bedeutung haben:
Q_{SS} = Plasmaspiegel im Fließgleichgewicht (steady state), in ng/ml;
J_{SS} = in vitro Hautflux, in µg/cm² x h;
A = wirksame Abgabefläche des Systems in cm²; und
Cl = Clearance (Reinigungsrate, Plasmamenge pro Zeiteinheit von einem bestimmten Wirkstoff) in 1/h; Wert für Vinpocetin entnommen aus Kobayashi, D. et al., Biol. Pharm. Bull., 16, (1993), 254-258).

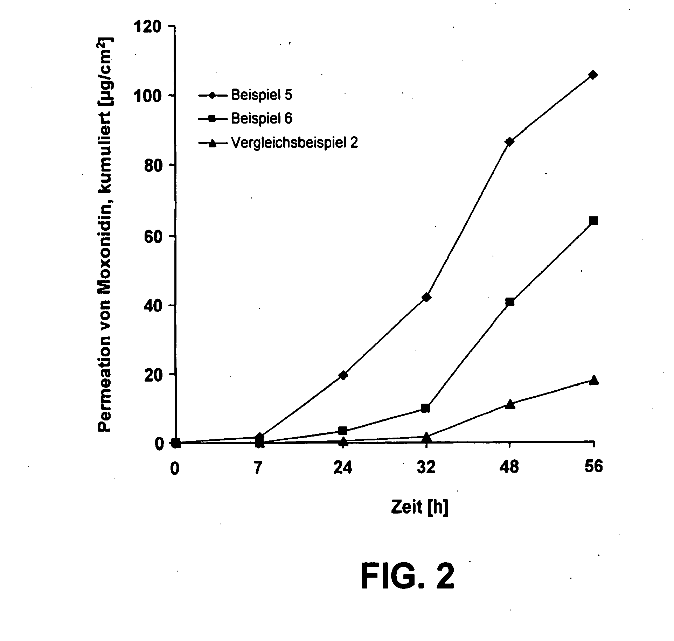

Die kumulierten Permeationsraten von Moxonidin werden in Figur 2,dargestellt, wobei die einzelnen Messpunkte den Mittelwert aus drei Einzelmessungen darstellen. Die Ergebnisse der Untersuchungen zur Permeation von Moxonidin sind in Tabelle 2 zusammengefasst.

Wie der Vergleich der Versuchsergebnisse zur transdermalen Freisetzung von Moxonidin zeigt, führt ein Flüssigreservoir-System gemäß Beispiel 6 zu Permeationsraten, die um etwa 60 % geringer sind als die mit einem Flüssigreservoir-System nach Beispiel 5 erzielten Permeationsraten. Figur 2 veranschaulicht den synergistischen Effekt der vier in einem Flüssigreservoir-System nach Beispiel 5 enthaltenen Enhancerkomponenten.

**Tabelle 2: Vergleich der mit verschiedenen transdermalen therapeutischen Systemen erzielten Permeationsraten von Moxonidin**

| System | Steuermembran | Haftkleber | Enhancer-Komponente (n) | Gew.-% der Komponenten im Enhancergemisch | In vitro-Hautflux [µg/cm²xh] |
|---|---|---|---|---|---|
| Beispiel 5 | Polyurethan | Polyacrylat | Miglyol Typ 812 | 35 | 2,79 |
| | | | Dimethylisosorbid | 25 | |
| | | | Eucalyptol | 25 | |
| | | | n-Dodecanol | 15 | |
| | | | | | |
| Beispiel 6 | Polyurethan | Polyacrylat | Lauryllactat | 35 | 1,56 |
| | | | Ölsäure | 25 | |
| | | | Eucalyptol | 25 | |
| | | | Menthol | 15 | |
| | | | | | |
| Vergleichs | | | | | 0,447 |
| beispiel 2 | | | | | |

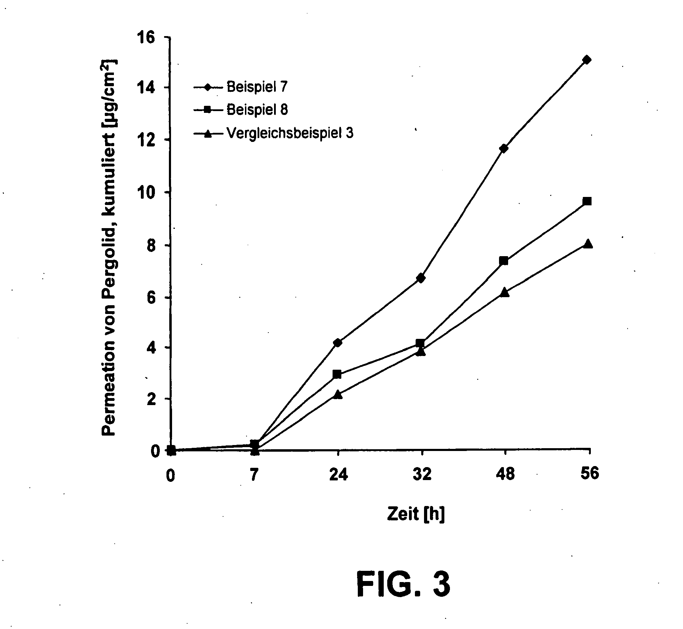

Die kumulierten Permeationsraten von Pergolid werden in Figur 3 dargestellt, wobei die einzelnen Messpunkte den Mittelwert aus drei Einzelmessungen darstellen. Die Ergebnisse der Untersuchungen zur Permeation von Moxonidin sind in Tabelle 3 zusammengefasst.

Wie der Vergleich der Versuchsergebnisse zur transdermalen Freisetzung von Pergolid zeigt, führt ein Flüssigreservoir-System gemäß Beispiel 8 zu Permeationsraten, die um etwa 60 % geringer sind als die mit einem Flüssigreservoir-System nach Beispiel 7, welches nur einen einzigen Permeationsenhancer enthält. Figur 3 veranschaulicht den synergistischen Effekt der vier in einem Flüssigreservoir-System nach Beispiel 7 enthaltenen Enhancerkomponenten im Vergleich zu dem Mono-Enhancersystem Eucalyptol (Bsp. 8).

**Tabelle 3: Vergleich der mit verschiedenen transdermalen therapeutischen Systemen erzielten Permeationsraten von Pergolid**

| System | Steuermembran | Haftkleber | Enhancer-Komponente (n) | Gew.-% der Komponenten im Enhancergemisch | In vitro-Hautflux [µg/cm²xh] |
|---|---|---|---|---|---|
| Beispiel 7 | Polyurethan | Polyacrylat | Miglyol Typ 812 | 35 | 0,309 |
| | | | Dimethylisosorbid | 25 | |
| | | | Eucalyptol | 25 | |
| | | | n-Dodecanol | 15 | |
| | | | | | |
| Beispiel 8 | Polyurethan | Polyacrylat | Eucalyptol | 100 | 0,185 |
| | | | | | |
| Vergleichs | | | | | 0,165 |
| beispiel 3 | | | | | |

Wie der Vergleich der Versuchsergebnisse zeigt, führen die erfindungsgemäßen Flüssigreservoir-Systeme zu Permeationsraten, die deutlich höher als die mit einem Matrix-TTS erzielbaren Permeationsraten sind und deutlich über dem gesetzten Ziel liegen.

## Patentansprüche

1. Transdermales therapeutisches System zur Verabreichung eines lipophilen, schlecht wasserlöslichen und wenig hautpermeablen Wirkstoffs, **dadurch gekennzeichnet, dass** der Wirkstoff in einem Mehrkomponenten-Enhancersystem gelöst oder suspendiert vorliegt, wobei das Enhancersystem aus 4 Komponenten besteht und jede Komponente des Mehrkomponenten-Enhancersystems aus einer anderen der Stoffgruppen
a) Terpene;
b) cyclische Glucitol-Ether;
c) mittelkettige Triglyceride aus Caprin- und Caprylsäure und/oder Linolsäure; und
d) längerkettige Alkohole mit einer Kettenlänge von 8 oder mehr Kohlenstoffatomen
ausgewählt ist.

2. Transdermales therapeutisches System nach Anspruch 1, **dadurch gekennzeichnet, dass** die Enhancer-Komponenten in einem Mischungsverhältnis vorliegen, bei dem der Anteil der am niedrigsten dosierten Komponente mindestens 10 Gew.-% und der Anteil der am höchsten dosierte Komponente höchstens 40 Gew.-% beträgt, bezogen auf das Mehrkomponenten-Enhancersystem.

3. Transdermales therapeutisches System nach Anspruch 1 oder 2, **dadurch gekennzeichnet, dass** der Wirkstoff aus der Gruppe ausgewählt ist, die Vinpocetin, Moxonidin, Pergolid und deren pharmazeutisch akzeptablen Salze umfasst.

4. Transdermales therapeutisches System nach einem der vorangehenden Ansprüche, **dadurch gekennzeichnet, dass** der Gehalt an Wirkstoff 0,1 bis 50 Gew.-%, vorzugsweise 5,0 bis 25,0 Gew.-%, beträgt, bezogen auf die wirkstoffhaltige Zubereitung.

5. Transdermales therapeutisches System nach einem der vorangehenden Ansprüche, **dadurch gekennzeichnet, dass** Wirkstoff und alle Enhancer-Komponenten in einem Flüssigreservoir vorliegen.

6. Transdermales therapeutisches System nach Anspruch 5, **dadurch gekennzeichnet, dass** das Flüssigreservoir eine Steuermembran umfasst, welche die Abgabe der Enhancerkomponenten und des Wirkstoffs kontrolliert.

7. Transdermales therapeutisches System nach Anspruch 6, **dadurch gekennzeichnet, dass** die Steuermembran aus einem Polymer besteht, das aus der Gruppe ausgewählt ist, die Polyethylene, Polypropylene, Silikone, Polyurethane und Copolymere aus Ethylen und Vinylacetat umfasst.

8. Transdermales therapeutisches System nach Anspruch 6, **dadurch gekennzeichnet, dass** das Flüssigreservoir-System eine Haftkleberschicht unter der Steuermembran umfasst, wobei der Haftkleber der Haftkleberschicht aus der Gruppe ausgewählt ist, die Silikone, Acrylate, Polyisobutylene und Copolymere aus Ethylen und Vinylacetat, letztere klebend gemacht unter Zusatz von Klebharzen, besteht.

9. Transdermales therapeutisches System nach Anspruch 8, **dadurch gekennzeichnet, dass** die Haftkleberschicht unter der Steuermembran die Freisetzung des Wirkstoffs kontrolliert, wobei sie ihre Steuerwirkung über die Schichtdicke ausübt.

10. Transdermales therapeutisches System nach Anspruch 8, **dadurch gekennzeichnet, dass** die Haftkleberschicht auf Basis von Copolymeren aus Ethylen und Vinylacetat mit Klebharzzusätzen ihre Steuerwirkung über das Verhältnis Ethylen zu Vinylacetat ausüben kann.

11. Transdermales therapeutisches System nach einem der vorangehenden Ansprüche, **dadurch gekennzeichnet, dass** die Wirkstoffzubereitung zusätzliche Hilfsstoffe im Sinne von Verdickungsmitteln enthält, die vorzugsweise aus der Gruppe ausgewählt sind, die Mineralöle, Wollwachse, Polyacrylsäuren, hochmolekulare Polyethylenglykole und feindisperses Siliziumdioxid enthält.

12. Transdermales therapeutisches System nach einem der vorangehenden Ansprüche, **dadurch gekennzeichnet, dass** die Wirkstoffzubereitung als Lösung, Dispersion, Suspension, Paste oder Gel vorliegt.

13. Transdermales therapeutisches System nach einem der vorangehenden Ansprüche, **dadurch gekennzeichnet, dass** die Wirkstoffzubereitung zusätzlich Steuerungselemente im Sinne einer verzögerten Abgabe enthält, die vorzugsweise aus der Gruppe ausgewählt sind, die Cyclodextrine, Polyvinylpyrrolidone und Cellulose-Derivate enthält.

14. Transdermales therapeutisches System nach einem der vorangehenden Anspruche, **dadurch gekennzeichnet, dass** sich das Mehrkomponenten-Enhancersystem wie folgt zusammensetzt:
a) 25 Gew.-% Eucalyptol,
b) 25 Gew.-% Dimethylisosorbid,
c) 35 Gew.-% Miglyol Typ 812, und
d) 15 Gew.-% 1-Dodecanol.

## Claims

1. Transdermal therapeutic system for administering a lipophilic, poorly water-soluble and sparingly skin-permeable active substance, **characterised in that** the active substance is present in dissolved or suspended form in a multi-component enhancer system, said enhancer system consisting of 4 components and each component of said multi-component enhancer system being selected from a different one of the substance groups:
a) terpenes;
b) cyclic glucitol ethers;
c) medium-chain triglycerides of capric and caprylic acid and/or of linoleic acid; and
d) longer-chain alcohols having a chain length of 8 or more carbon atoms

2. Transdermal therapeutic system according to claim 1, **characterised in that** the enhancer-components are present in a mixing ratio wherein the proportion of the component used in the lowest dose is at least 10%-wt. and the proportion of the most highly dosed component is maximally 40%-wt., relative to the multi-component enhancer system.

3. Transdermal therapeutic system according to claim 1 or 2, **characterised in that** the active substance is selected from the group comprising vinpocetine, moxonidine, pergolide and the pharmaceutically acceptable salts thereof.

4. Transdermal therapeutic system according to any one of the preceding claims, **characterised in that** the content of active substance is 0.1 to 50%-wt., preferably 5.0 to 25.0%-wt., relative to the active substance-containing preparation.

5. Transdermal therapeutic system according to any one of the preceding claims, **characterised in that** the active substance and all of the enhancer-components are present in a liquid reservoir.

6. Transdermal therapeutic system according to claim 5, **characterised in that** the liquid reservoir comprises a control membrane controlling the release of the enhancer components and of the active substance.

7. Transdermal therapeutic system according to claim 6, **characterised in that** the control membrane consists of a polymer selected from the group comprising polyethylenes, polypropylenes, silicones, polyurethanes and copolymers of ethylene and vinyl acetate.

8. Transdermal therapeutic system according to claim 6, **characterised in that** the liquid reservoir system comprises a pressure-sensitive adhesive layer located below the control membrane, wherein the pressure-sensitive adhesive of said pressure-sensitive adhesive layer is selected from the group consisting of silicones, acrylates, polyisobutylenes and copolymers of ethylene and vinyl acetate, the latter being rendered adhesive by addition of adhesive resins.

9. Transdermal therapeutic system according to claim 8, **characterised in that** the pressure-sensitive adhesive layer located below the control membrane controls the release of the active substance, said membrane exerting its controlling effect via its layer thickness.

10. Transdermal therapeutic system according to claim 8, **characterised in that** the pressure-sensitive adhesive layer based on copolymers of ethylene and vinyl acetate with addition of adhesive resins exerts its controlling effect via the ratio of ethylene to vinyl acetate.

11. Transdermal therapeutic system according to any one of the preceding claims, **characterised in that** the active substance preparation contains additional auxiliary substances, in the sense of thickening agents, which are preferably selected from the group containing mineral oils, wool waxes, polyacrylic acids, high-molecular polyethylene glycols and finely dispersed silicon dioxide.

12. Transdermal therapeutic system according to any one of the preceding claims, **characterised in that** the active substance preparation is present as a solution, dispersion, suspension, paste, or gel.

13. Transdermal therapeutic system according to any one of the preceding claims, **characterised in that** the active substance preparation additionally contains control elements in the sense of a retarded release, said control elements preferably being selected from the group containing cyclo-dextrins, polyvinyl pyrrolidones and cellulose derivatives.

14. Transdermal therapeutic system according to any one of the preceding claims, **characterised in that** the multi-component enhancer system is composed as follows:
a) 25%-wt. eucalyptol,
b) 25%-wt. dimethyl isosorbide,
c) 35%-wt. miglyol type 812, and
d) 15%-wt. 1-dodecanol.

## Revendications

1. Système thérapeutique transdermique pour l'administration d'une substance active lipophile, peu soluble dans l'eau et à faible perméabilité cutanée, **caractérisé en ce que** la substance active est présente en suspension ou à l'état dissous dans un système amplificateur à plusieurs composants, le système amplificateur étant constitué par 4 composants et chaque composant du système amplificateur à plusieurs composants étant choisi parmi un autre groupe de substances, à savoir :
a) des terpènes ;
b) des éthers cycliques de glucitol ;
c) des triglycérides à chaîne moyenne de l'acide caprique et caprylique et/ou de l'acide linoléique ; et
d) des alcools à longues chaînes, dont la longueur de chaîne comprend 8 atomes de carbone ou plus.

2. Système thérapeutique transdermique selon la revendication 1, **caractérisé en ce que** les composants amplificateurs sont présents en une proportion mélange dans laquelle la fraction du composant le plus faiblement dosé représente au moins 10 % en poids et la fraction du composant le plus fortement dosé s'élève au maximum à 40 % en poids, rapportés au système amplificateur à plusieurs composants.

3. Système thérapeutique transdermique selon la revendication 1 ou 2, **caractérisé en ce que** la substance active est choisie parmi le groupe qui comprend la vinpocétine, la moxonidine, le pergolide et leurs sels pharmaceutiquement acceptables.

4. Système thérapeutique transdermique selon l'une quelconque des revendications précédentes, **caractérisé en ce que** la teneur en substance active s'élève de 0,1 à 50 % en poids, de préférence de 5,0 à 25,0 % en poids, rapportés à la préparation contenant la substance active.

5. Système thérapeutique transdermique selon l'une quelconque des revendications précédentes, **caractérisé en ce que** la substance active et tous les composants amplificateurs sont présents dans un réservoir liquide.

6. Système thérapeutique transdermique selon la revendication 5, **caractérisé en ce que** le réservoir liquide comprend une membrane de réglage qui contrôle la libération des composants amplificateurs et de la substance active.

7. Système thérapeutique transdermique selon la revendication 6, **caractérisé en ce que** la membrane de réglage est constituée d'un polymère qui est choisi parmi le groupe qui contient des polyéthylènes, des polypropylènes, des silicones, des polyuréthanes et des copolymères d'éthylène et d'acétate de vinyle.

8. Système thérapeutique transdermique selon la revendication 6, **caractérisé en ce que** le système de réservoir liquide comprend une couche autoadhésive en dessous de la membrane de réglage, l'agent autoadhésif de la couche autoadhésive étant choisi parmi le groupe qui est constitué par des silicones, des acrylates, des polyisobutylènes et des copolymères d'éthylène et d'acétate de vinyle, ces derniers étant rendus adhésifs par addition de résines adhésives.

9. Système thérapeutique transdermique selon la revendication 8, **caractérisé en ce que** la couche autoadhésive en dessous de la membrane de réglage contrôle la libération de la substance active en exerçant son activité de réglage via l'épaisseur de la couche.

10. Système thérapeutique transdermique selon la revendication 8, **caractérisé en ce que** la couche autoadhésive, à base de copolymères d'éthylène et d'acétate de vinyle avec des additifs de résines adhésives, peut exercer son activité de réglage via le rapport de l'éthylène à l'acétate de vinyle.

11. Système thérapeutique transdermique selon l'une quelconque des revendications précédentes, **caractérisé en ce que** la préparation contenant une substance active contient des substances auxiliaires supplémentaires dans le sens d'agents épaississants qui sont choisis de préférence parmi le groupe qui contient des huiles minérales, des cires de laine, des acides polyacryliques, des polyéthylèneglycols à poids moléculaire élevé et du dioxyde de silicium finement dispersé.

12. Système thérapeutique transdermique selon l'une quelconque des revendications précédentes, **caractérisé en ce que** la préparation contenant une substance active est présente sous la forme d'une solution, d'une dispersion, d'une suspension, d'une pâte ou d'un gel.

13. Système thérapeutique transdermique selon l'une quelconque des revendications précédentes, **caractérisé en ce que** la préparation contenant une substance active contient en outre des éléments de réglage dans le sens d'une libération retardée, qui sont choisis de préférence parmi le groupe qui contient des cyclodextrines, des polyvinylpyrrolidones et des dérivés de la cellulose.

14. Système thérapeutique transdermique selon l'une quelconque des revendications précédentes, **caractérisé en ce que** le système amplificateur à plusieurs composants possède la composition ci-après :
a) de l'eucalyptol à concurrence de 25 % en poids ;
b) du diméthylisosorbide à concurrence de 25 % en poids ;
c) du Miglyol type 812 à concurrence de 35 % en poids; et
d) du 1-dodécanol à concurrence de 15 % en poids.
